Europäisches Patentamt

European Patent Office

Office européen des brevets

(11) Publication number: **0 183 421**
**B1**

# (12) EUROPEAN PATENT SPECIFICATION

(45) Date of publication of patent specification: **18.04.90**

(51) Int. Cl.⁵: **A 61 M 25/00, A 61 B 17/22**

(21) Application number: **85308191.7**

(22) Date of filing: **11.11.85**

(54) **Multi-procedural embolectomy catheter.**

(30) Priority: **15.11.84 US 671703**

(43) Date of publication of application:
**04.06.86 Bulletin 86/23**

(45) Publication of the grant of the patent:
**18.04.90 Bulletin 90/16**

(84) Designated Contracting States:
**BE CH DE FR GB IT LI NL SE**

(56) References cited:
**EP-A-0 125 844**
**FR-A-2 403 083**
**FR-A-2 520 236**
**GB-A-1 026 755**
**US-A-3 435 826**
**US-A-4 299 226**

(73) Proprietor: **Shiley Inc.**
**17600 Gillette Avenue**
**Irvine California (US)**

(72) Inventor: **Cottonaro, N. Cliff**
**2206, North Hathaway**
**Santa Ana California (US)**

(74) Representative: **Moore, James William, Dr. et al**
**Pfizer Limited Ramsgate Road**
**Sandwich Kent CT13 9NJ (GB)**

Courier Press, Leamington Spa, England.

## Description

The present invention relates to a multi-procedural embolectom catheter for visualization, isolation and subsequent removal of an arterial embolus, and infusion of sterile liquid for irrigation of the embolus site.

Embolectomy catheters, generally, are balloon catheters used to remove embolus. U.S. Patent No. 3,435,826 describes one example of an embolectomy catheter and discusses the use of embolectomy catheters to remove embolus in vessels and like passages in the body. In this embolectomy catheter, there is a first passage which inflates a sleeve to fix the embolus. A second passage discharges fluid behind the embolus to exert pressure upon it and move it toward the exit point. The disadvantage of this method is that while it removes the embolus, it does not provide for a washing out of the residual embolus debris associated with the removal. A further disadvantage is that the embolus located by this procedure is not visualized, but rather is located in such a blind fashion so as to possibly cause incomplete removal of the entire embolus.

U.S. Patent No. 4299226 describes a dual lumen balloon catheter continuously flushed with heparinised saline through an inner lumen, an outer lumen being used to inject dye and saline for inflating the balloon.

The catheter of the present invention has a distal infusion port for washing of the embolus site for removal of debris left behind after the embolus has been removed.

The infusion port also has an elastic covering with an opening in the covering such the catheter can hold its priming fluid when not in use but also is ready to infuse liquid for irrigation without lengthy delay. A further important feature is the ability to locate the embolus. The embolus can be located and visualized by means of radiopaque fluid and then removed without damaging the distressed vessel.

The catheter of the present invention combines all these features in one catheter. These features and other features discussed hereinafter, result in a catheter which is more efficient and also more economically produced. Further, the catheter of the present invention provides flexibility while providing for complete removal, of both the embolus and remaining debris, from the surgical site.

The present invention relates to a dual lumen embolectomy catheter comprising a flexible tube divided into first and second lumens. The distal end of the first lumen is provided with an aperture, and an elastic balloon is secured to the flexible tube at positions proximal and distal to the aperture. The proximal end of the first lumen is adapted for association with a means to inflate the elastic balloon through the aperture.

The second lumen is adapted at the proximal end with means to supply sterile liquid to the second lumen. The second lumen extends to a distal infusion port at the distal tip of the catheter.

The distal infusion port is provided with an elastic covering, which is secured to the catheter substantially enclosing the distal infusion port. The elastic covering has an opening preferably including a slit in the portion thereof overlying the distal infusion port. The opening is such that the flow of fluid through it is substantially greater out of than into the second lumen. The catheter tube of the present invention is preferably made of polyvinyl chloride and the elastic balloon and the elastic covering are preferably made of latex rubber.

Novel features and advantages of the present invention, in addition to those mentioned above, will become apparent from a reading of the following detailed description, given by way of example, in conjunction with the accompanying drawings wherein:

Fig. 1 is an overall view of an embolectomy catheter of the invention connected to a luer syringe, the catheter tube being broken to show both ends of the catheter in a simple figure;

Fig. 2 is a cross sectional view of an enlarged scale of the distal tip of the catheter of Fig. 1 taken on the line 22 looking in the direction of the arrows;

Fig. 3 is a cross sectional view taken on the line 33 in Fig. 1 looking in the direction of the arrows;

Fig. 4 is an end view of the catheter of Fig. 1;

Fig. 5 is a semi-schematic view of a catheter of the inventon positioned adjacent an embolus site showing the balloon inflated; and

Fig. 6 is a similar view to Fig. 5 showing the inflated balloon at a position beyond the embolus site.

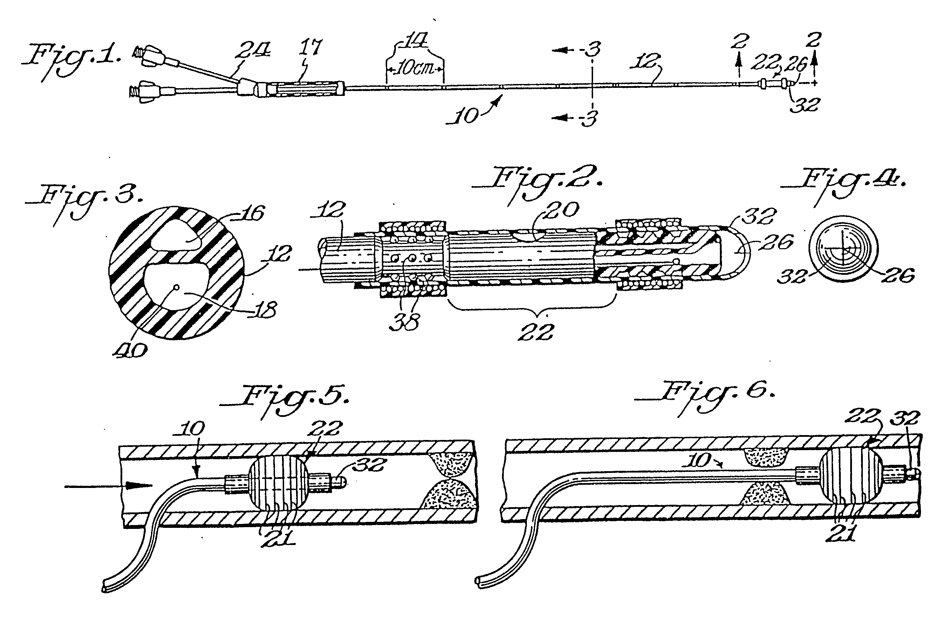

In Figs. 1 to 4 is illustrated an embolectomy catheter for infusion of sterile fluid, and isolation and removal of an arterial embolus. The catheter is externally smooth so that it may be easily inserted and pass through a blood vessel without trauma. The catheter, generally indicated at 10, is shown connected to a luer syringe, the latter not being part of the invention. The catheter 10 includes flexible tube 12 which is divided into first and second lumens 16, 18. The first lumen 16 is provided with an aperture 20 adjacent its distal end. The catheter 10 is further provided with an elastic balloon 22 which is secured to tube 12 at positions proximal and distal to the aperture 20.

Inflation of the elastic balloon 22 takes place through the aperture 20, located in the wall of the first lumen 16. The aperture is located from about 5 mm to 15 mm from the distal tip of the catheter. The proximal end of the first lumen 16 is adapted for association with an inflation source, generally indicated at 24. Preferably the inflation source 24 supplies sterile infusate to inflate the balloon 22.

The balloon 22 is made of highly elastic material, preferably latex rubber. Other suitable materials are polyurethane, and silicone rubber. In a preferred embodiment the elastic balloon 22 is ribbed with concentric rings 21 to augment the balloon symmetry and provide additional strength to the balloon body.

The second lumen 18 is adapted at the proximal end for association with means for connecting to a fluid source, generally indicated at 17. The second lumen 18 extends to a distal infusion port 32 at the distal tip of the tube 12. The distal infusion port 32 is provided with an elastic covering which encloses the distal end of the catheter, the elastic covering having an opening 26 which is in communication with the second lumen 18. The elastic covering is preferably made of latex rubber. Other suitable materials include polyurethane and silicone rubber.

In a preferred embodiment of the invention the elastic covering includes the elastic balloon 22 at the distal end of the tube 12. Preferably, the elastic covering is secured to the tube 12 by means of a series of wraps of suture material, most preferably a series of suture ties. The tube 12 also preferably includes rounded projections, generally indicated at 38, which serve several functions. The projections are made of X-ray opaque material, and, accordingly indicate the location of the distal infusion port 32 and the elastic balloon 22 when the catheter is in place in the vessel. Also the projections serve to make the attachment of the elastic covering to the distal end of the tube 12 more certain. The suture ties are preferably coated with biologically compatible epoxy resin.

The opening 26 in the elastic covering preferably has a slit configuration overlying the distal end of the catheter. More preferably, the slit configuration is a cross cut. Other suitable slit configurations are a single axis cut, double axis cuts, and a puncture or orifice cut.

The highly elastic nature of the covering prevents the retrograde flow of biological fluid in the vessel from entering the opening 26 in the elastic covering.

Additionally, the catheter 10 can be primed (i.e. can be filled completely with liquid) and can hold its prime until the liquid is needed to irrigate the debris after the embolus is removed. This feature of the catheter 10 eliminates the possibility of introducing an air embolus into the vessel during irrigation.

In a preferred embodiment, the catheter 10 also includes an integral guide wire 40. The guide wire is positioned in the second lumen 18 and provides a greater column rigidity to the catheter 10. The combination of the guide wire 40 with the soft, flexible, elastic covered tip adds the ability to manuver the catheter through a distressed vessel with a highly branched configuration without causing injury to the vessel. Yet such a combination allows the catheter to completely penetrate the emboli without buckling.

The tube 12 is flexible and generally elongated and is constructed of biologically compatible plastic having a diameter sufficiently small to permit it to be inserted in and advanced through body passages. The tube may, typically, have a outside body diameter of from about 1.00 mm to 2.33 mm. The overall length of the tube is preferably from about 40 cm to 100 cm. The tube 12 may be marked with indicia 14, preferably radiopaque graduated 10 centimeter markings along the tube length, to indicate the degree of insertion.

In use as shown in Fig. 5, the catheter 10 is inserted into the distressed vessel being treated and directed to the approximate location of the embolus site. When in position, the balloon 22 is inflated to occlude the distressed vessel. This step is critical because without occluding the vessel when the radiopaque dye is injected through the distal infusion port, the dye would leak out of the area and the embolus site would not be able to be visualized on the fluoroscope with any degree of accuracy.

After the embolus site is visually determined the balloon is deflated. As shown in Fig. 6, the catheter is then advanced through the embolus site and positioned at a point distal to the site. Sterile solution, preferably saline solution, is injected through the infusion port and the catheter, is simultaneously withdrawn with the balloon inflated, removing the embolus. The saline solution irrigates the embolus site and also maintains the physiological competency of the distressed blood vessel. After the embolus has been removed the site can be further irrigated to remove residual debris by injecting additional sterile solution.

The multi-procedural embolectomy catheter of the present invention eliminates many of the problems of previously used methods for removal of embolus. The present invention provides a disposable, relatively simple economical system for removal of embolus. The novel device of this invention has the unique advantage of providing multiple functions in one catheter:

(a) visualization of the embolus site,
(b) isolation of the embolus,
(c) removal of the embolus, and
(d) infusion of sterile liquid at the embolus site for irrigation.

In addition, the apparatus is of relatively uncomplicated design and offers easy manipulation for more accurate placement of the apparatus.

Further modifications will occur to those skilled in the art. The scope of the invention is defined by the appended claims and should not be understood as limited by the specific embodiments described herein.

**Claims**

1. A dual lumen embolectomy catheter (10) comprising:

a flexible tube (12) divided into first (16) and second lumens (18), an aperture (20) being provided adjacent the distal end of the first lumen (16) and being in communication with said first lumen, an elastic balloon (22), secured to the tube (12) at positions proximal and distal to the aperture (20), with the proximal end of the first lumen (16) being adapted for association with a means to inflate the elastic balloon (22) through the aperture;

the second lumen (18) being adapted at its proximal end with means to supply sterile liquid to the second lumen (18), the second lumen (18) extending to an infusion port (32) at the distal tip of said catheter (10);

characterised in that there is provided an elastic covering enclosing said infusion port (32) and secured to said catheter (10), said elastic covering having an opening (26), overlying the infusion port, said opening (26) being such that the flow of fluid through said opening (26) is substantially greater in the direction out of the second lumen (18) than into the second lumen.

2. The catheter (12) of claim 1 wherein the tube is made of polyvinyl chloride, the elastic balloon (22) is made of latex rubber and the elastic covering is made of latex rubber.

## Patentansprüche

1. Doppellumen-Embolektomiekatheter (10) umfassend:

eine biegsame Röhre (12), die in ein erstes (16) und zweites Lumen (18) unterteilt ist, eine Öffnung (20), die an der Peripherie des ersten Lumens (16) und in Verbindung mit diesem ersten Lumen vorgesehen ist, einen elastischen Ballon (22), der an der Röhre (12) proximal und distal von der Öffnung (20), befestigt ist, wobei das proximale Ende des ersten Lumens (16) zur Assoziation mit einem Mittel angepaßt ist, um den elastischen Ballon (22) durch die Öffnung aufzublasen;

wobei das zweite Lumen (18) an seinem proximalen Ende mit Mitteln angepaßt ist, um das zweite Lumen (18) mit steriler Flüssigkeit zu versorgen, und das zweite Lumen (18) sich zu einer Infusionsöffnung (32) am distalen Ende des Katheters (10) erstreckt,

dadurch gekennzeichnet, daß eine elastische Bedeckung, die die Infusionsöffnung (32) einschließt und an dem Katheter (10) angebracht ist, vorgesehen ist, wobei die elastische Bedeckung eine Öffnung (26) aufweist, die die Infusionsöffnung überdeckt und die Öffnung (26) derart ist, daß der Flüssigkeitsstrom durch diese Öffnung (26) wesentlich größer ist aus dem zweiten Lumen (18) hinaus als in das zweite Lumen hinein.

2. Katheter (12) nach Anspruch 1, worin die Röhre aus Polyvinylchlorid, der elastische Ballon (22) aus Latex-Gummi und die elastische Bedeckung aus Latex-Gummi bestehen.

## Revendications

1. Cathéter à embolectomie à double passage (10) comprenant:

— un tube flexible (12) divisé en un premier (16) et un second (18) passages, une ouverture (20) étant prévue au voisinage de l'extrémité distale du premier passage (16) et étant en communication avec ledit premier passage, un ballon élastique (22) fixé au tube (12) en des positions proximale et distale par rapport à l'ouverture (20), l'extrémité proximale dudit premier passage (16) étant adaptée a être associée à un moyen de gonflage du ballon élastique (22) au travers de l'ouverture;

— le second passage (18) étant adapté à son extrémité proximale à des moyens de fourniture de liquide stérile audit second passage (18), le second passage (18) s'étendant vers un orifice de perfusion (32) à l'extrémité distale dudit cathéter (10),

caractérisé en ce qu'il est prévu un recouvrement élastique renfermant ledit orifice de perfusion (32) et fixé audit cathéter (10), ledit recouvrement élastique ayant une ouverture (26) recouvrant l'orifice de perfusion, ladite ouverture (26) étant telle que l'écoulement du fluide par ladite ouverture (26) est sensiblement plus grande dans la direction de sortie du second passage (18) que d'entrée dans le second passage.

2. Cathéter (12) selon la revendication 1, dans lequel le tube est fait de poly(chlorure de vinyle), le ballon élastique (22) est fait de caoutchouc de latex et le recouvrement élastique de caoutchouc de latex.

Fig.1.

Fig.3.

Fig.2.

Fig.4.

Fig.5.

Fig.6.

EP 0 183 421 B1